# EUROPÄISCHE PATENTSCHRIFT

(11) **EP 0 935 966 B1**
(45) Veröffentlichungstag und Bekanntmachung des Hinweises auf die Patenterteilung: **12.12.2001**
(21) Anmeldenummer: 98810117.6
(22) Anmeldetag: 16.02.1998
(51) Int. Cl.: A61M 1/02, B01F 11/00

(54) **Verfahren und Vorrichtung zum Mischen von Antikoagulans und Frischblut**
Method and apparatus for mixing fresh blood and anticoagulant
Procédé et dispositif pour mélanger du sang frais et de l'anticoagulant

(43) Veröffentlichungstag der Anmeldung: 18.08.1999
(73) Patentinhaber: Möller Feinmechanik GmbH & Co., 36043 Fulda (DE)
(72) Erfinder: Heil, Markus, 36043 Fulda (DE)
(74) Vertreter: Riederer, Conrad A., Dr.

(56) Entgegenhaltungen:
- WO-A-95/04557
- DE-A- 19 529 872
- US-A- 5 238 304

## Beschreibung

Die vorliegende Erfindung betrifft ein Verfahren zum Mischen von in einem Blutbeutel befindlichem flüssigem Antikoagulans mit während einer Blutentnahme in den Blutbeutel einfliessendem Frischblut, bei welchem der Beutel wenigstens zeitweise bewegt wird.

Die Erfindung betrifft auch eine Vorrichtung zum Mischen von in einem Blutbeutel befindlichem flüssigem Antikoagulans mit während einer Blutentnahme in den Blutbeutel einfliessendem Frischblut, insbesondere für Blutwaagen, mit einer Aufnahmeeinrichtung für den Blutbeutel, wenigstens einer Achse und wenigstens einem Antrieb zum Bewegen der Aufnahmeeinrichtung, und mit einer Steuerungseinrichtung zur Steuerung der Bewegung.

Zur Mischung von Frischblut mit einem Antikoagulans in einem Beutel während der Blutentnahme werden in Blutwaagen Schaukeleinrichtungen mit einer flachen Aufnahmeschale zur Aufnahme des Blutbeutels verwendet. Diese Aufnahmeschale vollführt regelmässig und periodisch ein gleichbleibendes Bewegungsmuster. Sie führt beispielsweise während etwa 4 Sekunden zwei komplette Schaukelbewegungen aus, um anschliessend für etwa 2,5 Sekunden in horizontaler Lage still zu stehen, bevor der Vorgang wiederholt wird.

Im deutschen Gebrauchsmuster G 94 10 237.6 wird eine Vorrichtung zum Mischen von Frischblut mit einem flüssigen Antikoaguians in einem Beutel offenbart, bei welcher die Aufnahmeschale auf zwei Vertikalzapfen drehbar gelagert ist, welche Zapfen auf je einer Exzenterscheibe angeordnet sind, um synchron zu wirken und mit der Aufnahmeschale eine horizontale Kreis- bzw. Taumelbewegung auszuführen. Der Antrieb des Exzenters erfolgt über ein Reibrad. Diese Ausführung lässt wenig Spielraum für eine Anpassung der Bewegung.

Mit diesen regelmässigen, gleichbleibenden und periodischen Bewegungen des Beutels werden das in den Beutel einlaufende Frischblut und das sich im Beutel befindliche Antikoagulans in Bewegung gehalten und durchmischt.

Es hat sich gezeigt, dass mit solchen kontinuierlichen periodischen Bewegungen in den allermeisten Fällen mit je nach Anwender unterschiedlich grosser Häufigkeit eine genügende Durchmischung von Antikoagulans und Frischblut zustandekommt. Die Durchmischung ist aber nicht bei allen Anwendern immer genügend, so dass, wenn auch in weniger als 1 Prozent der Blutentnahmen, eine Klumpenbildung festgestellt werden muss.

Aus der US-A-5238304 ist ein Verfahren und ein Mischer zum Mischen unterschiedlicher Festkörper bekannt. Da Festkörper bezüglich Grösse, Gestalt oder Dichte unterschiedlich sein können, haben sie bei regelmässigen Mischbewegungen die Tendenz, sich zu entmischen. Eine solche Entmischung ist unvorhersehbar und kann jederzeit von neuem stattfinden.

Um mit einfachen Mitteln eine im Vergleich zum Stand der Technik gleichmässigere, gründliche Mischung von unterschiedlichen Festkörpern viel rascher erreichen zu können, ohne dass im Behälter tote Zonen auftreten, wird in dieser Schrift vorgeschlagen, dass ein Mischbehälter um zwei Achsen rotiert wird, wobei die Rotationsgeschwindigkeit um eine der Achsen stochasitschen Schwankungen unterliegt, so dass der Behälter eine durchgehend zufällige unregelmässige Mischbewegung mit immerwährend zufällig wechselnder Froudezahl ausführt.

Die WO-A-9504557 beschreibt ein Blutspende-Überwachungsgerät mit einer Plattform zur Aufnahme eines Blutbeutels, welche schwenkbar auf einem Traggestell angeordnet ist. Um die Plattform zu schaukeln ist ein Antrieb vorgesehen. Das Gerät ist mit einer Wägeeinrichtung zum Wägen der auf der Plattform vorliegenden Blutmenge und mit einer Klemme zum Unterbrechen des Blutzuflusses ausgerüstet. Eine Steuereinheit erfasst die Daten der Wägeeinrichtung und überwacht die Änderung des Gewichts des Blutbeutels. Abhängig von der Änderung des Gewichts aktiviert die Steuereinheit die Klemme. Wenn also eine vorgewählte Menge Blut in den Beutel geflossen ist, so wird der Blutzufluss unterbrochen.

Das in den Beutel einströmende Blut kommt im Beutel in Kontakt mit einem dort vorliegenden Antikoagulans. Damit eine gute Durchmischung von Antikoagulans und Blut gewährleistet ist, wird die Plattform nach Zufluss einer bestimmten Menge Bluts (z.B. 10 g) geschaukelt. Die Schaukelbewegung wird danach in regelmässigen Abständen identisch wiederholt. Die Schaukelbewegung wird erreicht durch eine rotierende Scheibe, in die ein zentral unter der Plattform angeordneter Stift exzentrisch eingreift. Die Plattform ist über zwei senkrecht zueinander liegende Achsen kippbar und führt während einer Rotation der Scheibe eine jedesmal identische Schwenkbewegung um die beiden Achsen aus. Eine Veränderung der Bewegung ist nicht vorgesehen.

Mit dieser Mischbewegung werde, so die Schrift, das Blut während einer durchschnittlichen Spendezeit von sechs Minuten rasch mit dem Antikoagulans durchmischt. Dabei würden nur geringe Ablagerungen von Blutbestandteilen wie roten Blutkörperchen auftreten.

Es ist deshalb Aufgabe der Erfindung ein Durchmischungsverfahren und eine Mischvorrichtung bereitzustellen, mit welchen die Gerinselbildung weiter verringert werden kann.

Erfindungsgemäss wird dies dadurch erreicht, dass bei einem Verfahren der genannten Gattung das räumliche und/oder zeitliche Bewegungsmuster des Beutels in Abhängigkeit wenigstens eines sich während der Entnahme verändernden Parameters verändert wird. Dadurch gelangen über die Dauer einer Entnahme unterschiedliche Bewegungsmuster zur Anwendung, welche auf die besonderen Gegebenheiten, wie z.B. in den Beutel eingelaufene Blutmenge, Einlaufgeschwindigkeit des Blutes, verstrichene Zeit seit Beginn der Blutentnahme, Rücksicht nehmen können.

Vorzugsweise wird der Beutel über zwei im Wesentlichen senkrecht zueinander stehende Achsen bewegt, wodurch eine Durchmischung des Blutes und des Antikoagulans in zwei senkrecht zueinander liegenden Richtungen erreicht wird. Diese Bewegungen über diese Achsen können sowohl Schwenkbewegungen als auch translatorische Bewegungen sein. Dabei ist es möglich, dass bei horizontaler erster Achse die zweite Achse ebenfalls horizontal oder aber vertikal angeordnet ist. Abhängig davon, ob die Bewegungen Schwenk- und/oder translatorische Bewegungen sind und abhängig von der Ausrichtung der Achsen sind unterschiedliche Bewegungsmuster erreichbar. Vorteilhaft wird der Beutel sogar über drei im Wesentlichen senkrecht zueinander angeordneten Achsen bewegt. Dadurch ist die Anzahl möglicher unterscheidbarer Bewegungsmuster nochmals erhöht.

Zweckmässigerweise werden die Schwenkbewegungen um jede der Achsen bzw. die translatorischen Bewegungen auf jeder Achse individuell gesteuert, so dass unbegrenzt viele Bewegungsmuster erreicht werden können. Die Steuerung dieser Muster erfolgt dabei den heutigen Möglichkeiten entsprechend z.B. durch einen programmierbaren Prozessor. Die Ansprüche 5 bis 7 führen einzelne bevorzugte Bewegungsabläufe über nur eine Schaukelachse genauer aus. Wenn auch sehr einfache Bewegungsmuster wie die dort erwähnten möglich sind, so kann die Bewegung des Beutels aber auch viel komplexer, ja sogar sowohl zeitlich wie räumlich eine chaotische sein. Eine Solche könnte beispielsweise durch einen Zufallsgenerator gesteuert sein.

Bei einer Vorrichtung der erwähnten Gattung sind erfindungsgemäss Mittel zur Veränderung eines räumlichen und/oder zeitlichen Bewegungsmusters in Abhängigkeit von Messwerten vorgesehen.

Eine solche Vorrichtung weist vorteilhaft wenigstens einen zweiten Antrieb auf, um zusätzlich zu einer Schwenkbewegung um die Achse die Aufnahmeeinrichtung auf der Achse zu verschieben. Vorteilhaft weist die Vorrichtung eine zweite Achse senkrecht zur ersten Achse und wenigstens einen weiteren Antrieb auf, um die Aufnahmeeinrichtung um diese zweite Achse zu verschwenken und/oder auf dieser zweiten Achse zu verschieben. Diese Achsen können beide etwa waagerecht ausgerichtet sein oder aber eine davon senkrecht. Je nachdem sind andere Bewegungsmuster mit dem Beutel durchführbar.

Ist jedoch eine dritte, in einer Grundstellung zu den zwei anderen senkrecht angeordnete Achse und wenigstens ein weiterer Antrieb vorgesehen, um die Aufnahmeeinrichtung um diese dritte Achse zu verschwenken und/oder zu verschieben, so sind praktisch alle erdenklichen Bewegungsmuster ausführbar. Zweckmässigerweise ist dazu eine individuelle Ansteuerung der einzelnen Antriebe mit der Steuerungseinheit vorgesehen.

Bei mehreren Achsen, über welche die Aufnahmeeinrichtung bewegt werden kann, sind unterschiedliche Anordnung der Achsen möglich. Je nach Ausrichtung der Achsen und Verwendung der Achsen als translatorische und/oder Schwenkachsen sind unterschiedliche Bewegungsmuster möglich. Dabei spielt die Ausrichtung der Aufnahmeeinrichtung bzw. des Beutels bezüglich der Achsen ebenfalls eine Rolle für die Bewegungsmöglichkeiten, welche durch die verschiedenen Achsen erreicht werden. Mit drei im Wesentlichen senkrecht zueinander ausgerichteten Achsen können unabhängig der spezifischen Anordnung der Achsen neben den translatorischen Schüttelbewegungen in den drei Richtungen eine Drehbewegung in der horizontalen Ebene und Kippbewegungen in zwei senkrecht zueinander angeordneten senkrechten Ebenen ausgeführt werden. Daher ist mit einer solchen Achsanordnung z.B. die einfache und bekannte Kipp- oder Schaukelbewegung ebenso ausführbar wie Taumelbewegungen ähnlich der Exzenterbewegung aus dem Gebrauchsmuster 94 10 237.6, aber auch kompliziertere bis hin zu chaotischen Bewegungsabläufen sind möglich. Die Steuerung der Bewegungsabläufe aufgrund von Messdaten, z.B. der verstrichenen Zeit, des Gewichts bzw. der Blutmenge der Blutentnahme, der Gewichtszunahme der Blutentnahme pro Zeiteinheit oder allenfalls auch der Konzentration oder Gleichmässigkeit der Konzentration des Antikoagulans im Blut, lässt die Bewegungsabläufe auch zeitlich optimieren.

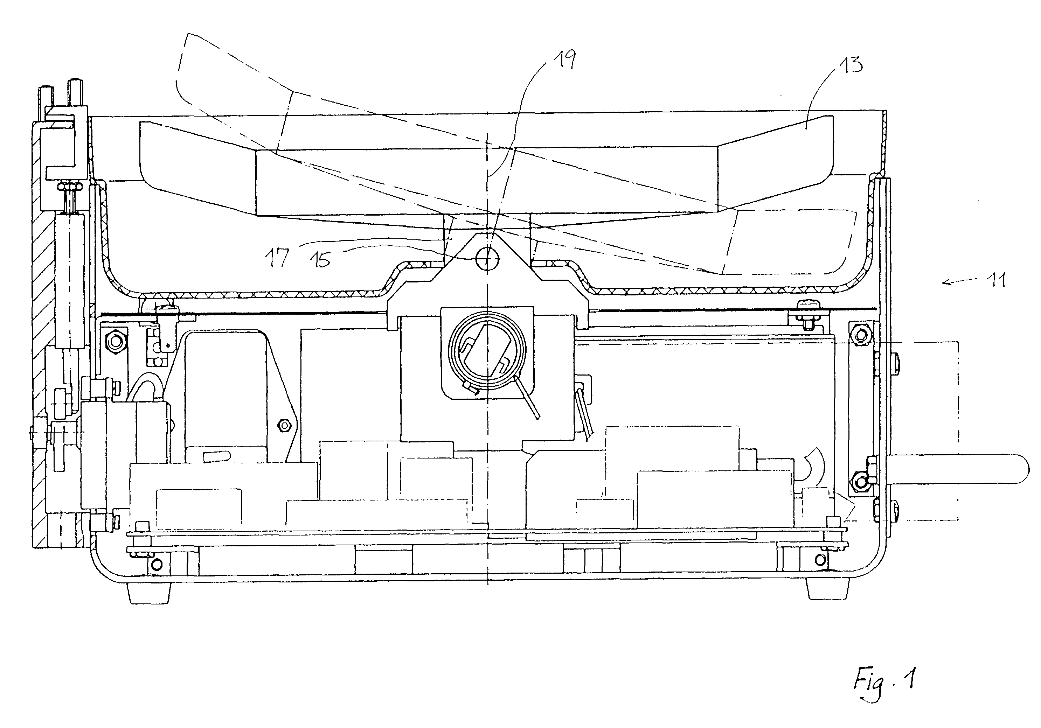

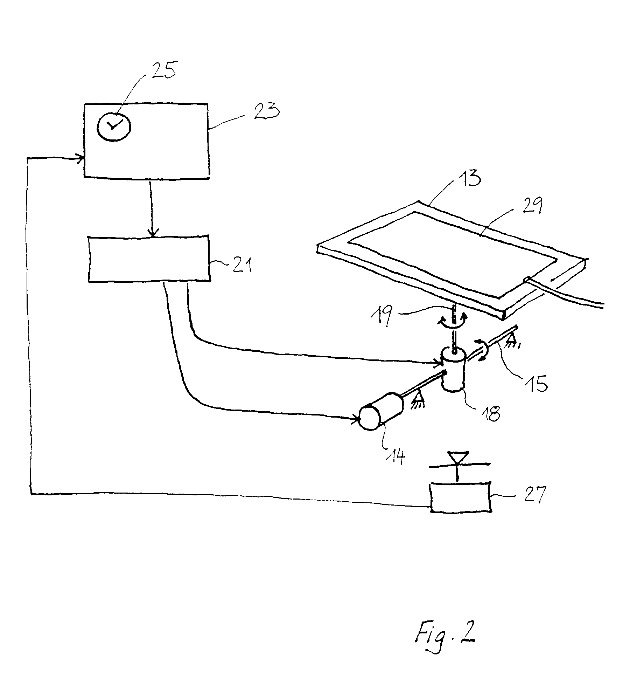

Nachfolgend wird ein Ausführungsbeispiel und eine beispielhafte Funktionsweise der Erfindung unter Bezugnahme auf die Figuren 1 und 2 beschrieben:
Figur 1 zeigt eine erfindungsgemässe Blutwaage 11 mit einer flachen Aufnahmeschale 13 für einen Blutbeutel.
Figur 2 zeigt eine schematische Zeichnung der im Ausführungsbeispiel von Figur 1 verwendeten Vorrichtung zur Mischung von Antikoagulans und Frischblut.

Die Aufnahmeschale 13 ist wie bei bekannten Blutwaagen mit einem Antrieb 14 um eine Achse 15 verschwenkbar. Auf der Achse 15 sitzt ein Klotz 17, auf welchem die Aufnahmeschale 13 angeordnet ist. In diesem Klotz 17 ist ein Antrieb 18 vorgesehen, um die Aufnahmeschale 13 um eine mit der Schale 13 verschwenkbare Achse 19 senkrecht zur Achse 15 und senkrecht zur Ebene der Schale 13 zu drehen. Mit einer Steuereinrichtung 21 wird die Bewegung der Schale 13 gesteuert, indem beide Antriebe 14,18 einzeln angesteuert werden. Ein programmierter Prozessor 23 gibt dazu der Steuereinrichtung 21 die notwendigen Impulse. Der Prozessor 23 verfügt über eine Zeitmessung 25. Im Programm wird die verstrichene Zeit seit Beginn der Blutentnahme berücksichtigt, um die Bewegung der Schale 13 zeitlich und räumlich festzulegen. Beispielsweise wird frühestens nach einer bestimmten Zeit mit der allgemein üblichen Schaukelbewegung mit Pausen zwischen zwei Schaukelbewegungen begonnen.

Ebenso verwertet das Programm Informationen über das Gewicht der Blutentnahme, wobei Gewicht und Volumen in direktem, praktisch konstantem Verhältnis stehen. Dazu ist der Prozessor 23 mit einer Wägeeinrichtung 27 verbunden. Beispielsweise wird bei zu geringer in den Beutel 29 eingelaufener Blutmenge nach der erwähnten Zeitspanne eine Schaukelbewegung verhindert und eine oszillierende Schwenkbewegung um die Achse 19 veranlasst.

Bei einer Blutentnahme wird beispielsweise zu Beginn die Schale 13 in Schrägstellung gehalten, so dass das einlaufende Frischblut unten im Beutel 29 direkt in das angesammelte Antikoagulans einfliesst. Nach zwei Minuten oder bei 50 ml Blut beginnt die Schale eine oszillierende Hin- und Herbewegung um die Achse 19 mit einer Periode von etwa einer Sekunde auszuführen. Nach 5 Minuten oder 100 ml eingelaufenem Frischblut beginnt eine Schaukelbewegung um die Achse 15. Dabei wird durch Verdrehen der Schale 13 um die Achse 19 abwechslungsweise über die eine oder andere Diagonale der Schale 13 geschaukelt.

Durch entsprechende Programmwahl kann mit der selben Waage aber auch ein Bewegungsablauf wie folgt aussehen: Zu Beginn der Blutentnahme schaukelt die Waage öfter (wählbar zwischen 2 und 40 mal) hintereinander ohne Pause. Danach, z.B. nach Einlaufen von 100 ml, beginnt beispielsweise die bekannte Schaukelbewegung mit Pausen dazwischen. Es kann auch programmiert werden, dass die Schale 13 zwischen den ersten Schaukelbewegungen eine Pause in einer Schräglage, z.B. abwechselnd nach links und rechts geneigt, einlegt, um später die gewöhnliche Schaukelbewegung mit horizontaler Ruhestellung auszuführen.

Durch entsprechend abgestimmte Ansteuerung der Antriebe 14,18 kann auch eine kreisende Bewegung des Beutelinhaltes ausgelöst werden. Eine solche Bewegung wird beispielsweise ab einen Beutelinhalt von 200 ml zwischen Schaukelbewegungen eingefügt, um die Gleichmässigkeit der Durchmischung von Blut und Antikoagulans zu verbessern.

Die angeführten Bewegungsabläufe sind als einzelne aus einer unendlichen Fülle von Möglichkeiten gegriffen. Durch die Verwendung von Messwerten zur Bestimmung des anzuwendenden Bewegungsmusters lassen sich die Bewegungsabläufe vielmehr in beliebiger Vielfalt gestalten, wobei auf die Zweckmässigkeit des Ablaufes in Bezug auf die Verbesserung der Mischung zu achten ist.

Ähnliche Bewegungsabläufe lassen sich auch gestalten, wenn die Aufnahmeschale 13 nicht um die Achse 19 verdrehbar ist, sondern auf der Achse 15 translatorisch verschiebbar gelagert ist und durch einen Antrieb, beispielsweise ebenfalls im Klotz 17, hin und her verschoben wird. Ebenso wird durch eine Verschwenkung der Achse 15 um eine dazu normale und horizontal liegende Achse Ähnliches erreicht. Wesentlich ist, dass die Abfolge von Bewegungen oder Bewegungsmustern durch eine Steuerung des Antriebs verändert werden kann, wobei die Zeit, das Gewicht oder Volumen der in den Beutel eingelaufenen Blutmenge und/oder andere Messwerte dazu genutzt werden, die Bewegung oder das Bewegungsmuster zu bestimmen. Welche Bewegungen, welche Bewegungsmuster und welche Abläufe geeigneter sind als andere, ist je nach Anwender unterschiedlich und muss im einzelnen eruiert werden.

Zusammenfassend kann gesagt werden, dass bei einer Blutentnahme zum Mischen von in einem Beutel 29 vorliegendem Antikoagulans mit in den Beutel 29 einfliessendem Frischblut der Beutel 29 zusammen mit einer Aufnahmeeinrichtung 13 für den Beutel 29 bewegt wird. Diese Bewegung erfolgt jedoch nicht über den ganzen Zeitraum der Blutentnahme in einem gleichbleibenden Rhythmus und/oder nach einem gleichbleibenden Bewegungsmuster. Aufgrund von Messdaten wie verstrichene Zeit und Gewicht der Blutentnahme werden durch einen programmierbaren Prozessor 23 nacheinander unterschiedliche Bewegungssequenzen ausgelöst. Durch Softwareanpassungen wird dieser Bewegungsablauf verschiedenen Bedürfnissen angepasst. Eine erfindungsgemäss ausgerüstete Waage verfügt neben eines entsprechenden Prozessors 23 und der angemessenen Steuereinrichtung 21 vorteilhaft über mehrere Antriebe 14,18 und allenfalls über mehr als eine Achse 15,19. Um diese Achsen und/oder auf diesen Achsen 15,19 wird die Aufnahmeeinrichtung 13 für den Blutbeutel 29 durch die Antriebe 14,18 verschwenkt bzw. verschoben. Dabei sind die Antriebe für die einzelnen Richtungen und Bewegungen unabhängig voneinander ansteuerbar.

## Patentansprüche

1. Verfahren zum Mischen von in einem Blutbeutel (29) befindlichem flüssigem Antikoagulans mit während einer Blutentnahme in den Blutbeutel (29) einfliessendem Frischblut, bei welchem der Beutel wenigstens zeitweise nach einem Bewegungsmuster bewegt wird, **dadurch gekennzeichnet, dass** das räumliche und/oder zeitliche Bewegungsmuster des Beutels (29) in Abhängigkeit wenigstens eines sich während der Entnahme verändernden Parameters verändert wird.

2. Verfahren nach Anspruch 1, **dadurch gekennzeichnet, dass** der Beutel (29) über zwei im Wesentlichen senkrecht zueinander stehende Achsen (15,19) bewegt wird.

3. Verfahren nach Anspruch 1, **dadurch gekennzeichnet, dass** der Beutel (29) über drei im Wesentlichen senkrecht zu einander angeordnete Achsen bewegt wird.

4. Verfahren nach Anspruch 2 oder 3, **dadurch gekennzeichnet, dass** jede Schwenkbewegung und jede translatorische Bewegung des Beutels (29) individuell gesteuert ist.

5. Verfahren nach einem der Ansprüche 1 bis 4, **dadurch gekennzeichnet, dass** eine Schwenkbewegung, insbesondere zu Beginn einer Blutentnahme, mehr als 2 mal ausgeführt wird, vorzugsweise eine wählbare Anzahl mal, z.B. 3 bis 40 mal, bevor sie angehalten wird.

6. Verfahren nach einem der Ansprüche 1 bis 5, **dadurch gekennzeichnet, dass** die Beutelbewegung, insbesondere bis zu einer gewissen eingeflossenen Blutmenge, z.B. 100 ml, in einer Schräglage des Beutels (29) angehalten wird.

7. Verfahren nach einem der Ansprüche 1 bis 6, **dadurch gekennzeichnet, dass** zu Beginn der Blutentnahme der Beutel (29) in Schräglage gelagert wird, wobei der Einlauf des Frischblutes im unteren Bereich erfolgt, so dass das Frischblut direkt in das Antikoagulans einfliesst.

8. Vorrichtung zum Mischen von in einem Blutbeutel (29) befindlichem flüssigem Antikoagulans mit während einer Blutentnahme in den Blutbeutel (29) einfliessendem Frischblut, insbesondere für eine Blutwaage (11), mit einer Aufnahmeeinrichtung (13) für den Blutbeutel (29), wenigstens einer Achse (15), wenigstens einem Antrieb (14) zum Bewegen der Aufnahmeeinrichtung (13), und mit einer Steuerungseinrichtung (21) zur Steuerung der Bewegung, **gekennzeichnet durch** Mittel (23,25,27) zur Veränderung des räumlichen und/oder zeitlichen Bewegungsmusters in Abhängigkeit von Messwerten.

9. Vorrichtung nach Anspruch 8, **gekennzeichnet durch** einen zweiten Antrieb, um zusätzlich zu einer Schwenkbewegung um die Achse (15) die Aufnahmeeinrichtung auf der Achse (15) zu verschieben.

10. Vorrichtung nach Anspruch 8 oder 9, **gekennzeichnet durch** eine zweite Achse (19) senkrecht zur ersten Achse (15) und wenigstens einen weiteren Antrieb (18), um die Aufnahmeeinrichtung (13) um diese zweite Achse (19) zu verschwenken und/oder auf dieser zweiten Achse zu verschieben.

11. Vorrichtung nach Anspruch 10, **gekennzeichnet durch** eine dritte, in einer Grundstellung zu den zwei anderen senkrecht angeordnete Achse und wenigstens einen weiteren Antrieb, um die Aufnahmeeinrichtung um diese dritte Achse zu verschwenken und/oder auf dieser dritten Achse zu verschieben.

12. Vorrichtung nach einem der Ansprüche 8 bis 11, **gekennzeichnet durch** eine individuelle Ansteuerung der einzelnen Antriebe.

## Claims

1. Method for mixing liquid anticoagulant located in a blood pouch (29) with fresh blood flowing into the said blood pouch (29) during a withdrawal of blood, in which method the pouch is moved, at least intermittently, according to a pattern of movement, **characterised in that** the spatial and/or chronological pattern of movement of the pouch (29) is varied in dependence upon at least one parameter which varies during the withdrawal.

2. Method according to claim 1, **characterised in that** the pouch (29) is moved via two spindles (15, 19) which are essentially perpendicular to one another.

3. Method according to claim 1, **characterised in that** the pouch (29) is moved via three spindles which are disposed essentially perpendicularly to one another.

4. Method according to claim 2 or 3, **characterised in that** each swivelling movement and each translatory movement of the pouch (29) is individually controlled.

5. Method according to one of claims 1 to 4, **characterised in that** a swivelling movement, particularly at the beginning of a withdrawal of blood, is executed more than twice, preferably a selectable number of times, for example 3 to 40 times, before it is stopped.

6. Method according to one of claims 1 to 5, **characterised in that** the movement of the pouch is stopped with the said pouch (29) in an oblique position, particularly until a certain quantity of blood which has flowed in, for example 100 ml, is reached.

7. Method according to one of claims 1 to 6, **characterised in that**, at the beginning of the withdrawal of blood, the pouch (29) is mounted in an oblique position, the running-in of the fresh blood taking place in the lower region so that the said fresh blood flows directly into the anticoagulant.

8. Device, particularly for a blood-weighing machine (11), for mixing liquid anticoagulant located in a blood pouch (29) with fresh blood flowing into the said blood pouch (29) during a withdrawal of blood, the said device having a holding apparatus (13) for the blood pouch (29), at least one spindle (15), at least one drive (14) for moving the said holding apparatus (13) and a control apparatus (21) for controlling the movement, **characterised by** means (23, 25, 27) for varying the spatial and/or chronological pattern of movement in dependence upon measured values.

9. Device according to claim 8, **characterised by** a second drive for the purpose of displacing the holding apparatus on the spindle (15) in addition to a swivelling movement about the said spindle (15).

10. Device according to claim 8 or 9, **characterised by** a second spindle (19) perpendicular to the first spindle (15) and at least one further drive (18) for the purpose of horizontally swinging the holding apparatus (13) about the said second spindle (19) and/or displacing it on the said second spindle.

11. Device according to claim 10, **characterised by** a third spindle which, in a basic position, is disposed perpendicularly to the two other spindles, and at least one further drive for the purpose of horizontally swinging the holding apparatus about the said third spindle and/or displacing it on the said third spindle.

12. Device according to one of claims 8 to 11, **characterised by** individual activation of the individual drives.

## Revendications

1. Procédé destiné à mélanger un anticoagulant liquide, contenu dans une poche de prélèvement de sang (29), avec le sang frais, affluant dans la poche (29) pendant un prélèvement de sang, dans lequel procédé la poche est agitée au moins temporairement selon un modèle de mouvements, **caractérisé en ce que** le modèle de mouvements dans l'espace et/ou dans le temps, effectué par la poche (29), varie en fonction d'au moins un paramètre variant au cours du prélèvement.

2. Procédé selon la revendication 1, **caractérisé en ce que** la poche (29) est mise en mouvement par l'intermédiaire de deux axes (15, 19) sensiblement perpendiculaires l'un à l'autre.

3. Procédé selon la revendication 1, **caractérisé en ce que** la poche (29) est mise en mouvement par l'intermédiaire de trois axes sensiblement perpendiculaires l'un à l'autre.

4. Procédé selon la revendication 2 ou 3, **caractérisé en ce que** chaque mouvement oscillant et chaque mouvement de translation de la poche (29) peuvent être commandés individuellement.

5. Procédé selon l'une quelconque des revendications 1 à 4, **caractérisé en ce qu'**un mouvement oscillant, en particulier au début du prélèvement de sang, est effectué plus de 2 fois, de préférence un nombre de fois à choisir, entre 3 et 40 fois par exemple, avant d'être stoppé.

6. Procédé selon l'une quelconque des revendications 1 à 5, **caractérisé en ce que** le mouvement de la poche, en particulier jusqu'à qu'une quantité déterminée de sang prélevé, 100 ml par exemple, est stoppé lorsque la poche (29) est en position inclinée.

7. Procédé selon l'une quelconque des revendications 1 à 6, **caractérisé en ce que**, au début du prélèvement de sang, la poche (29) est posée en position oblique, le sang frais affluant dans la partie inférieure, de telle sorte que le sang frais afflue directement dans l'anticoagulant.

8. Dispositif destiné à mélanger un anticoagulant liquide, contenu dans une poche de prélèvement de sang (29), avec le sang frais, affluant dans la poche (29) pendant un prélèvement de sang, en particulier pour une balance de prélèvement sanguin (11), comprenant un dispositif de réception (13) pour la poche de prélèvement (29), au moins un axe (15) et au moins un moteur (14) destiné à mettre en mouvement le dispositif de réception (13), et un dispositif de commande (21) destiné à commander le mouvement, **caractérisé par** des moyens (23, 25, 27) destinés à varier le modèle de mouvement dans l'espace et/ou dans le temps en fonction des valeurs de mesure.

9. Dispositif selon la revendication 8, **caractérisé par** un deuxième moteur, permettant d'appliquer sur le dispositif de réception non seulement un mouvement oscillant autour de l'axe (15), mais aussi un mouvement de coulissement sur l'axe (15).

10. Dispositif selon la revendication 8 ou 9, **caractérisé par** un deuxième axe (19) perpendiculaire au premier axe (15) et au moins un moteur supplémentaire (18), destiné à faire tourner le dispositif de réception (13) autour de ce deuxième axe (19) et/ou à le faire coulisser sur ce deuxième axe.

11. Dispositif selon la revendication 10, **caractérisé par** un troisième axe, dont la position de base est perpendiculaire aux deux autres axes, et au moins un moteur supplémentaire destiné à faire tourner le dispositif de réception autour de ce troisième axe et/ou à le faire coulisser sur ce troisième axe.

12. Dispositif selon l'une quelconque des revendications 8 à 11, **caractérisé par** une commande individuelle de chaque moteur.
